# EUROPÄISCHE PATENTSCHRIFT

(11) **EP 1 652 479 B1**
(45) Veröffentlichungstag und Bekanntmachung des Hinweises auf die Patenterteilung: **20.02.2008**
(21) Anmeldenummer: 04025566.3
(22) Anmeldetag: 27.10.2004
(51) Int. Cl.: A61B 17/02

(54) **Wirbelspreizinstrument mit Markern**
Spine distractor with markers
Ecarteur vertébral avec des indicateurs de position

(43) Veröffentlichungstag der Anmeldung: 03.05.2006
(73) Patentinhaber: BrainLAB AG, 85551 Kirchheim/Heimstetten (DE)
(72) Erfinder: Reed, Pete, Cincinatti OH 45236 (US); Schaffrath, Claus, 81377 München (DE); Kleinszig, Gerhard, 85656 Buch (DE); Becht, Gunther, 85609 Dornach (DE)
(74) Vertreter: Engelhard, Maximilian

(56) Entgegenhaltungen:
- EP-A- 1 454 589
- WO-A-03/092507
- US-A1- 2003 236 447
- US-B1- 6 340 363
- US-B1- 6 739 068

## Beschreibung

Die vorliegende Erfindung betrifft ein Wirbelspreizinstrument zum auseinander Bringen bzw. Spreizen zweiter Wirbel einer Wirbelsäule mittels zwei Spreizfingern. Bevorzugt ist das Wirbelspreizinstrument außerdem ausgebildet, um eine künstliche Bandscheibe zwischen die gespreizten Wirbel einzubringen.

Ein Wirbelspreizinstrument ist beispielsweise aus US 6,478,800 B1 bekannt. Mittels zweier Finger (Bezugszeichen 50A und 50B) werden zwei Wirbel gespreizt. Eine künstliche Bandscheibe (Bezugszeichen 24) wird mittels einer Schiebestange (Bezugszeichen 20) in den durch das Spreizen geschaffenen Freiraum zwischen den beiden Fingern hindurch hineingeschoben. Insbesondere wird dort eine mögliche Ausführungsform eines Spreizmechanismus beschrieben. Der Spreizmechanismus arbeitet so, dass die zwei Spreizfinger Verlängerungen jeweils eines Hebelarms sind, die um ein gemeinsames Lager schwenkbar sind. Die künstliche Bandscheibe ist zwischen den beiden Hebelarmen eingeklemmt und befindet sich distal des Lagers. Die Hebelarme verringern ihren Abstand in distaler Richtung. Der Abstand zwischen den Hebelarmen wird also umso kleiner, je näher sie sich an der Wirbelsäule befinden. Wird die distale Bandscheibe nun in distale Richtung weiter verschoben, so drängt sie dabei die beiden Hebelarme auseinander, was zu einem Spreizen von zwei Wirbeln der Bandscheiben führt, so dass letztendlich die künstliche Bandscheibe in einen Freiraum zwischen den beiden Wirbeln durch die Spreizfinger hindurch geschoben werden kann. Der Freiraum zwischen den Wirbeln ergibt sich insbesondere durch die vorab durchgeführte chirurgische Entfernung der beschädigten Bandscheibe.

Aus US 6,261,296 B1 ist ein Wirbelspreizinstrument bekannt. Das Wirbelspreizinstrument weist einen scherenartigen Wirbelspreizmechanismus auf. Dabei sind zwei Schenkel, wie bei einer Schere um einen Bolzen drehbar angeordnet. Die proximalen Handgriffe laufen in proximaler Richtung auseinander. Durch Zusammendrücken der Handgriffe werden die Spreizfinger am distalen Ende des scherenförmigen Spreizinstruments auseinander bewegt, während sich die Schenkel um den Bolzen drehen. Der Abstand zwischen den Spreizfingern ist am proximalen Ende des Spreizinstruments durch eine Stellschraube einstellbar, die die beiden Handgriffe am proximalen Ende der Schere verbindet.

Aus US 6,736,068 B1 ist ein Spreizinstrument bekannt, an dem eine Skala vorgesehen ist, um die Weite der Spreizung zu bestimmen.

Aus WO 03092507 ist ein Instrument zum Separieren von Wirbeln bekannt. Eine Anzeige ist vorgesehen, um die Position eines Betätigungsglieds relativ zu einem ersten und zweiten Glied anzuzeigen.

Aufgabe der Erfindung ist es, ein Wirbelspreizinstrument und ein Instrumentennavigationssystem mit dem erfindungsgemäßen Wirbelspreizinstrument zu schaffen, das eine genaue Kontrolle des Spreizvorgangs oder Spreizzustandes erlaubt.

Vorstehende Aufgabe ist durch den Gegenstand der Ansprüche 1 und 8 gelöst. Vorteilhafte Weiterbildungen gehen aus den Unteransprüchen hervor.

Erfindungsgemäß dient das Wirbelspreizinstrument vorzugsweise dem Auseinanderbringen oder Spreizen zweier Wirbel einer Wirbelsäule mittels zwei Spreizfingern. Die Spreizfinger sind vorzugsweise Bestandteil eines Spreizmechanismus, der dem Verstellen des Abstandes zwischen den Spreizfingern dient. Bei den Spreizfingern handelt es sich vorzugsweise um das distale Ende des Spreizmechanismus. Vorzugsweise sind die Spreizfinger scheibenförmig oder keilförmig ausgebildet.

Der Spreizmechanismus umfasst einen ersten Teil und einen zweiten Teil. Die Teile sind in ihrer Eigenschaft der Gestalt, dass sie ihre Relativpositionen zueinander ändern, wenn der Abstand zwischen den Spreizfingern geändert wird. Vorzugsweise ist die Änderung ihrer Relativpositionen eine Funktion der Abstandsänderung der Spreizfinger, so dass aus einer bestimmten Veränderung der Relativposition der Teile auf eine Abstandsänderung der Spreizfinger geschlossen werden kann. Vorzugsweise ist der Spreizmechanismus so gestaltet, dass aus der Relativposition der Teile auf den absoluten Abstand zwischen den Spreizfingern geschlossen werden kann. Vorzugsweise ist die Relativposition also eine Funktion des Abstandes der Spreizfinger. Anders ausgedrückt sind den verschiedenen relativen Positionen, die der erste und der zweite Teil zueinander einnehmen können, vorzugsweise jeweils ein vorbestimmter Abstand oder eine Relativposition der beiden Spreizfinger zueinander eindeutig (bijektiv) zugeordnet.

Am ersten Teil ist mindestens ein Marker angebracht und am zweiten Teil ist mindestens ein Marker angebracht. Der erste und zweite Teil werden so gewählt, dass die Veränderung ihrer relativen Positionen zueinander eine Funktion der Abstandsänderung sind. Vorzugsweise ist also jeder relativen Position zwischen dem ersten und zweiten Teil ein Abstand oder eine relative Position zwischen den beiden Spreizfingern eindeutig (bijektiv) zugeordnet. Vorzugsweise ist eine Änderung der relativen Positionen zwischen dem ersten und zweiten Teil eine Funktion der Abstandsänderung zwischen den beiden Spreizfingern.

Durch die vorgenannte Anordnung ist es möglich, durch Detektion der Position des ersten und zweiten Markers auf den Abstand zwischen den Spreizfingern zu schließen. Dabei wird vorzugsweise die bekannte Geometrie des Wirbelspreizinstruments genutzt. Vorzugsweise wird der Abstand zwischen den Spreizfingern basierend auf den bekannten relativen Positionen bzw. Abständen zwischen den Spreizfingern und den Markern berechnet. Insbesondere ist die Beziehung zwischen den verschiedenen relativen Positionen des ersten und zweiten Markers zu den verschiedenen Abständen vorbekannt (z.B. als Tabelle oder mathematische Funktion).

Am Wirbelspreizinstrument sind mindestens zwei weitere Marker vorgesehen. Dadurch wird es erlaubt, durch Detektion der Marker nicht nur den Abstand der Spreizfinger, sondern auch die absolute Position der Spreizfinger in den von Kamera beobachteten Raum zu bestimmen. Dazu spannen vorzugsweise drei der insgesamt somit mindestens vier Marker eine Ebene auf. Die insgesamt mindestens vier Marker spannen vorzugsweise einen dreidimensionalen Raum auf. Vorzugsweise ist ein Referenzstern vorgesehen, der drei der mindestens vier Marker umfasst. Vorzugsweise ist dieser Referenzstern am ersten Teil angebracht, wobei vorzugsweise die Positionsbeziehung zwischen den beiden Spreizfingern und dem ersten Teil vorbekannt ist, um somit auf die absolute Position der zwei Spreizfinger im Raum durch Detektion des Referenzsterns zu schließen. Insbesondere kann durch die Detektion des Referenzsterns die Bewegung des Wirbelspreizinstruments im Raum und somit insbesondere bei der Operation verfolgt werden. Vorzugsweise ist insbesondere der Abstand der Marker des Referenzsterns zu den Spreizfingern bei einem vorgegebenen Abstand der Spreizfinger, insbesondere Abstand 0, vorbekannt. Die mindestens drei Marker des Referenzsterns haben vorzugsweise eine ebenfalls bekannte und feststehende Relativposition, die sich insbesondere nicht durch den Spreizvorgang ändert.

Alternativ können auch beispielsweise zwei der Marker am ersten Teil mit feststehender vorgegebener Positionsbeziehung und zwei der Marker am zweiten Teil mit feststehender vorgegebener Positionsbeziehung vorgesehen sein. Auch auf diese Art und Weise kann die Position des Wirbelspreizinstruments und der Spreizfinger im Raum verfolgt werden und der Abstand der Spreizfinger bestimmt werden.

Bei den Markern handelt es sich vorzugsweise um so genannte passive Marker, die Detektionsstrahlen oder Detektionswellen reflektieren. Bei den Detektionsstrahlen oder Detektionswellen handelt es sich insbesondere um elektromagnetische Strahlung, wie sichtbares Licht und Infrarotlicht oder UV-Licht. Bei den Detektionswellen handelt es sich insbesondere um Schallwellen, wie z.B. Ultraschall.

Bei den Detektoren handelt es sich um geeignete Detektoren für die Detektionsstrahlen und Detektionswellen, also insbesondere um Kameras, die Licht (UV, sichtbares Licht, Infrarotlicht) messen können, oder um beispielsweise Schalldetektoren. Die Detektoren, insbesondere Kameras sind dabei vorzugsweise so angeordnet, dass sie sämtliche am Wirbelspreizinstrument angebrachten Marker (d.h. die von ihnen ausgehenden Detektionsstrahlen bzw. - wellen) erfassen können und ihre Position im Raum durch Auswertung der Detektionsergebnisse exakt bestimmen können.

Anstelle der passiven Marker können auch so genannte aktive Marker treten, die vorgenannten Detektionsstrahlen oder Detektionswellen aktiv emittieren. Die vorgenannten Kameras stellen Beispiele für Detektionsmittel zur Detektion der Detektionsstrahlen bzw. -wellen dar.

Bei der Erfindung zur Anwendung kommende Marker, Referenzsterne und Navigationssysteme sind beispielsweise in DE 195 36 180 A1, DE 296 23 941 U1 und DE 196 39 615 A1 offenbart, die US 5,769,861, US 6,351,659 B1 und US 2002-095081 A1 entsprechen.

Insgesamt sind also mindestens vier Marker vorgesehen, um den Abstand der Spreizfinger und die Lage des Wirbelspreizinstruments im Raum zu bestimmen. Vorzugsweise ist mindestens einer der Marker proximal von den Spreizfingern angeordnet. Vorzugsweise gilt dies für alle Marker. Besonders vorzugsweise sind die Marker möglichst am proximalen Ende des Wirbelspreizinstruments angeordnet, um die Bewegungsfreiheit am distalen Ende des Wirbelspreizinstruments nicht zu beeinträchtigen. Vorzugsweise ist der erste Teil an einem Teil oder Abschnitt des Wirbelspreizinstruments (des Spreizmechanismus) angeordnet, der bei Betätigung des Spreizmechanismus zum Verstellen des Abstands nicht bewegt wird, das heißt keine Lageänderung auf Grund des Spreizvorganges erfährt. Anders ausgedrückt ist die Position des ersten Teils vorzugsweise unabhängig vom Abstand der Spreizfinger und wird nicht durch die Änderung des Abstands beeinflusst. Beispielsweise befindet sich der erste Teil des Spreizmechanismus an einem Abschnitt, der als Drehgelenk ausgebildet ist, wobei sich um das Drehgelenk zwei Schenkel drehen, an deren Ende sich die Spreizfinger jeweils befinden. Auch kann der erste Teil ortsfest bezüglich dieses Drehgelenks sein, d.h. seine Lage diesbezüglich nicht ändern. Insbesondere stellt vorzugsweise der erste Teil einen Abschnitt dar, um den sich die Spreizfinger drehen. Auch ist der erste Teil vorzugsweise ortsfest bezüglich eines geometrischen Schwer- oder Mittelpunktes der Spreizfinger.

Der zweite Teil ist vorzugsweise proximal vom ersten Teil angebracht. Dadurch beeinträchtigen die Marker den zu operierenden Bereich nicht. Der zweite Teil ist vorzugsweise ein Teil, der eine Lageänderung erfährt, wenn sich der Abstand zwischen den Spreizfingern ändert, insbesondere ist der zweite Teil mechanisch bewegungsgekoppelt mit mindestens einem der Spreizfinger verbunden oder damit einstückig. Handelt es sich um eine Spreizung, bei der zwei Schenkel um einen Drehpunkt gedreht werden, so ist vorzugsweise der zweite Teil in einem vom Drehpunkt proximalen Abschnitt angeordnet, wobei der zweite Teil eine Drehung um einen Winkel erfährt, die dem Winkel der Drehung eines Spreizfingers um den Drehpunkt entspricht oder eine Funktion davon ist.

Vorzugsweise ist der erste Teil ein Teil des Spreizmechanismus oder des Wirbelspreizinstruments, der einen Arm, an dem sich der erste Spreizfinger befindet, mit einem Arm, an dem sich der zweite Spreizfinger befindet, verbindet. Beispielsweise kann der zweite Teil nicht nur ein Drehgelenk sein, sondern bei einer anderen Ausführungsform ein Hebelstützpunkt, der als Stützpunkt für eine Wippbewegung zweier Schenkel dient, an deren Ende sich jeweils ein Spreizfinger befindet, wie dies beispielsweise in US 6,478,800 beschrieben ist. Die Schenkel wippen dabei um den Hebelstützpunkt.

Auch kann der Wirbelspreizmechanismus scherenartig, wie z.B. in US 6,261,296 B1 beschrieben, ausgebildet sein.

Auch kann der Spreizmechanismus in der Art eines Wagenhebers ausgebildet sein, bei dem zwei Schenkel durch ein Drehkreuz verbunden sind, wobei sich jeweils an einem Ende der Schenkel einer der Spreizfinger befindet. Durch Verdrehen eines Gewindes werden gemäß dem Wagenheberprinzip die beiden Schenkel auseinander oder zusammen gefahren (insbesondere parallel auseinander oder zusammen gefahren). Der erste Teil befindet sich insbesondere an Abschnitten, die durch Drehen des Gewindes ihre Lage nicht ändern. Der erste Teil ist also beispielsweise am Gewindelager angeordnet oder diesbezüglich ortsfest oder bezüglich eines geometrischen Mittelpunktes der beiden Spreizfinger ortsfest. Der zweite Teil kann ein Abschnitt sein, der mit Drehung des Gewindes wandert, also sich relativ zum geometrischen Mittelpunkt der beiden Spreizfinger bewegt. Auch in diesem Fall ist der zweite Teil vorzugsweise proximal des Mittelspunkts des Drehkreuzes oder des ersten Teils angeordnet. Der erste Teil ist vorzugsweise an einem Gehäuse angeordnet, bezüglich dem das Gewinde drehbar gelagert ist, während der zweite Teil bei Drehung des Gewindes relativ zum Gehäuse wandert. Vorzugsweise ist der zweite Teil so ausgebildet, dass er sich linear, also geradlinig, bewegt, wobei die zurückgelegte Strecke eine Funktion des Abstandes der Spreizfinger ist.

Vorzugsweise ist das Wirbelspreizinstrument so ausgebildet, dass es einen Scheibenhalter aufweist, um eine künstliche Bandscheibe zu halten. Der Scheibenhalter kann separat von den Spreizfingern ausgebildet sein und getrennt von den Spreizfingern beweglich sein, wie dies beispielsweise aus US 6,478,800 bekannt ist, oder mindestens einer der beiden Spreizfinger, bevorzugt beide Spreizfinger, sind als Scheibenhalter ausgebildet.

Im folgenden wird die bevorzugte Ausführungsform beschrieben, wonach die Spreizfinger als Scheibenhalter ausgebildet sind. Hierzu sind beispielsweise Vertiefungen in den Spreizfingern vorgesehen, in die eine künstliche Bandscheibe passgenau eingebracht werden kann und durch Presspassung hält, wenn die beiden Spreizfinger einen vorgegebenen Abstand einnehmen. Die künstliche Bandscheibe wird also klemmend zwischen den beiden Spreizfingern gehalten. Da auf Grund der Marker die Position der Spreizfinger bekannt ist, ist auch bekannt, wo sich die künstliche Bandscheibe befindet. Somit wird das Einbringen der künstlichen Bandscheibe zwischen zwei Wirbeln einer Wirbelsäule für den Praktiker erleichtert.

Alternativ oder zusätzlich kann auch eine separate Scheibenhalterung vorgesehen sein, die insbesondere auch im gespreizten Zustand der Finger die künstliche Bandscheibe hält. Diese separate Scheibenhalterung ist vorzugsweise so ausgebildet, dass sie eine Verlagerung der Bandscheibe in distale Richtung erlaubt. Vorzugsweise ist die separate Scheibenhalterung so ausgebildet, dass die Scheibe linear in einer Ebene verlagerbar ist, die in der Mitte zwischen den gespreizten Spreizfingern sich befindet. Beispielsweise ist hierzu eine Lenkstange vorgesehen, die mittig zwischen den Schenkeln angeordnet ist, an deren Ende sich die Spreizfinger befinden. Vorzugsweise ist an dem separaten Scheibenhalter ebenfalls ein Marker angebracht, um die Position der künstlichen Bandscheibe auf Grund des bekannten Abstands zwischen dem Ende, an dem sich die künstliche Bandscheibe befindet, und dem Marker bestimmen zu können. Dabei ist der separate Scheibenhalter vorzugsweise nur in einer relativ zum Wirbelspreizinstrument bekannten Richtung beweglich, um so die absolute Position der künstlichen Bandscheibe bestimmen zu können.

Das erfindungsgemäße Wirbelspreizinstrument wird vorzugsweise als Bestandteil eines Instrumentennavigationssystems eingesetzt. Dieses umfasst vorzugsweise Kameras oder andere Strahlendetektionsmittel, um die Position der Marker im dreidimensionalen Raum bestimmen zu können. Sind passive Marker vorgesehen, so sind vorzugsweise weiter Mittel zum Aussenden oder Abstrahlen der Detektionsstrahlen oder -wellen, insbesondere Beleuchtungsmittel angeordnet, die die passiven Marker bestrahlen, insbesondere beleuchten, so dass diese die Strahlen zu den Detektionsmitteln (insbesondere Kameras oder Lichtsensoren) reflektieren können. Weiter ist ein Computer vorzugsweise vorgesehen, um aus der detektierten Strahlung die Position der Marker im dreidimensionalen Raum zu berechnen.

Im folgenden werden Ausführungsformen der Erfindung beschrieben. Dabei werden weitere erfindungswesentliche Merkmale offenbart. Merkmale verschiedener Ausführungsformen können miteinander kombiniert werden.
- Fig. 1a: zeigt ein Schema für die Anordnung von Markern an einem erfindungsgemäßen Wirbelspreizinstrument;
- Fig. 1b und 1c: zeigen schematisch die Funktion eines Spreizmechanismus;
- Fig. 2a, 2b und 2c: zeigen verschiedene Ansichten eines erfindungsgemäßen Wirbelspreizinstruments;
- Fig. 3: zeigt eine perspektivische Ansicht einer anderen Ausführungsform eines erfindungsgemäßen Wirbelspreizinstruments.

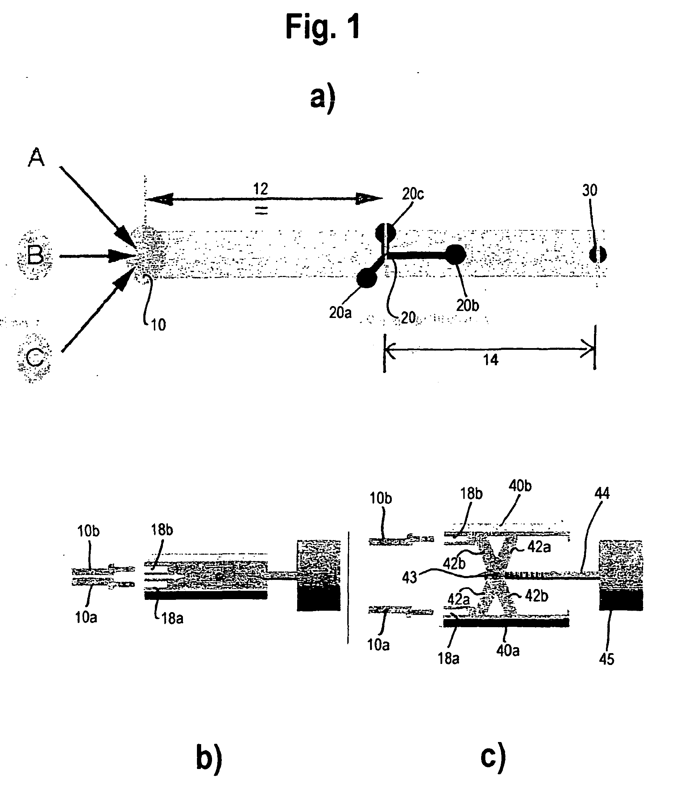

Fig. 1a zeigt das Prinzip der Markeranordnung bei einem erfindungsgemäßen Wirbelspreizinstrument. Die Spitze mit den Spreizfingern ist schematisch bei 10 gezeigt. Bei 10 können verschiedene künstliche Bandscheiben A, B und C angebracht werden. Vorzugsweise sind die Spreizfinger 10 so ausgebildet, dass verschiedene künstliche Bandscheiben A, B oder C anbringbar sind, um so möglichst flexibel das Wirbelspreizinstrument verwenden zu können. Die Spreizfinger 10 befinden sich in einem festen Abstand 12 von dem Referenzstern 20 entfernt, der drei Markerkugeln 20a, 20b und 20c aufweist. Ein weiterer Marker ist mit 30 bezeichnet. Er befindet sich in einem variablen Abstand 14 zum Referenzstern 20. Der Abstand 14 variiert mit dem Abstand der Spreizfinger 10.

Fig. 1b und 1c zeigen eine Ausführungsform eines erfindungsgemäßen Spreizmechanismus, der nach dem Wagenheberprinzip arbeitet.

Fig. 1b zeigt Spreizfinger 10a und 10b, die in dazu passende Ausnehmungen 18b und 18a lösbar einsetzbar sind. Auf diese Art und Weise können verschiedene Spreizfinger passend zu den gewünschten Anforderungen verwendet werden. Insbesondere können verschiedene Spreizfinger passend zu der einzusetzenden künstlichen Bandscheibe gewählt werden.

Wie in Fig. 1c gezeigt ist, sind die Ausnehmungen 18b und 18a jeweils mit zwei Schenkeln 40b und 40a verbunden, die parallel auseinander gefahren werden können. Ein Drehkreuz 42 mit Armen 42a und 42b ist mit den Schenkeln 40a und 40b verbunden. Beispielsweise ist der Arm 42b drehbar mit dem Schenkel 40b verbunden und kann in einer Schiene im Schenkel 40a in Längsrichtung des Instruments gleiten. Entsprechend ist beispielsweise der Arm 42a drehbar mit dem Schenkel 40a verbunden und kann in einer Schiene im Schenkel 40b gleiten. Die Arme 42a und 42b sind durch ein Drehlager 43 drehbar miteinander verbunden. Mit dem Drehgelenk 43 ist ein Ende der Gewindestange 44 verbunden. Die Gewindestange 44 dreht sich in einem Innengewinde des Gehäuses 45 und ist relativ zum Gehäuse 45 dadurch in Längsrichtung verlagerbar. Das Innengewinde dient also als Lagerung für die Gewindestange. Die Schenkel 40a und 40b haben durch einen Abstandshalter (nicht gezeigt) einen vorgegebenen Abstand zum Gehäuse 45 und können sich nicht in Längsrichtung bewegen, sondern nur aufeinander zu und voneinander weg. Wird nun das Drehgelenk 43 vom Gehäuse weg bewegt, so werden so die Schenkel 40a und 40b auseinander bewegt. Wird das Drehgelenk 43 durch das Gewinde 44 zum Gehäuse hin gezogen, so bewegen sich die Schenkel 40a und 40b aufeinander zu. Das Gehäuse ist bezüglich eines geometrischen Mittelpunktes der beiden Spreizfinger ortsfest und ändert insbesondere seine Lage nicht durch Änderung des Abstandes der Spreizfinger. Das Gehäuse dient als erster Teil des Wirbelspreizinstruments.

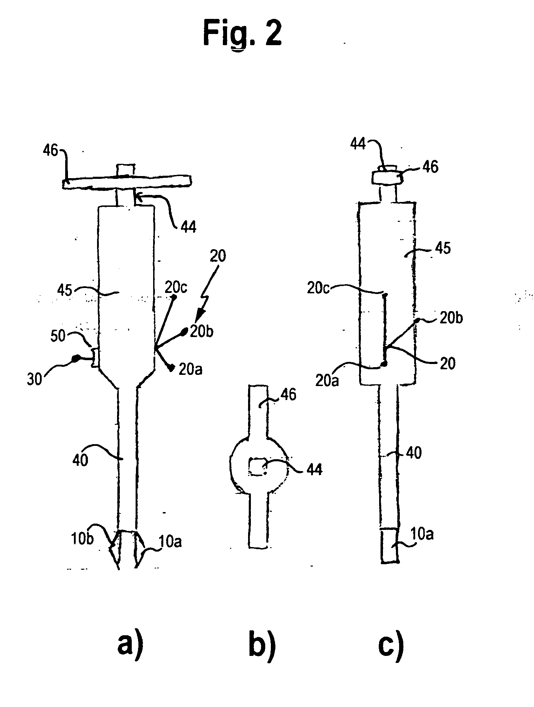

Fig. 2 zeigt eine Ausführungsform, die den Mechanismus der Fig. 1b und 1c verwendet. Gleiche Bezugszeichen der Fig. 2 bezeichnen dieselben Teile wie in Fig. 1.

Die Schenkel 40a und 40b sind generell mit 40 bezeichnet, da sie sich im aneinander anliegenden Zustand befinden. Am proximalen Ende des Gehäuses 45 tritt das Gewinde 44 hervor. Durch einen Handgriff 46, der drehfest mit dem Gewinde 44 verbunden ist, lässt sich das Gewinde 44 im Gehäuse 45 drehen, so dass es in Längsrichtung des Gehäuses wandert. Dadurch werden die Schenkel 40 auseinander getrieben, wie zuvor bezüglich der Fig. 1b und 1c erläutert wurde. Mit der Gewindestange 40 ist eine Anzeige 50 verbunden, an der der Marker 30 angebracht ist. Die Anzeige 50 dient als zweiter Teil des Wirbelspreizinstruments und kann in einem nicht gezeigten Spalt des Gehäuses wandern. Die Anzeige 50 ist mit der Gewindestange 44 so verbunden, dass sie deren Längsbewegung mitmacht. Die Position der Anzeige 50 im nicht gezeigten Spalt spiegelt somit den Abstand der Spreizfinger 10a und 10b wider. Gegenüber liegend zur Anzeige 50 mit dem Marker 30 befindet sich der Referenzstern 20 mit den Markern 20a, 20b und 20c. Der Referenzstern 20 ist mit dem Gehäuse 45 fest verbunden und wandert somit nicht mit der Drehbewegung der Gewindestange 44.

Fig. 2b zeigt eine Ansicht von dem proximalen Ende des Wirbelspreizinstruments.

Fig. 2c zeigt eine Seitenansicht der Fig. 2a.

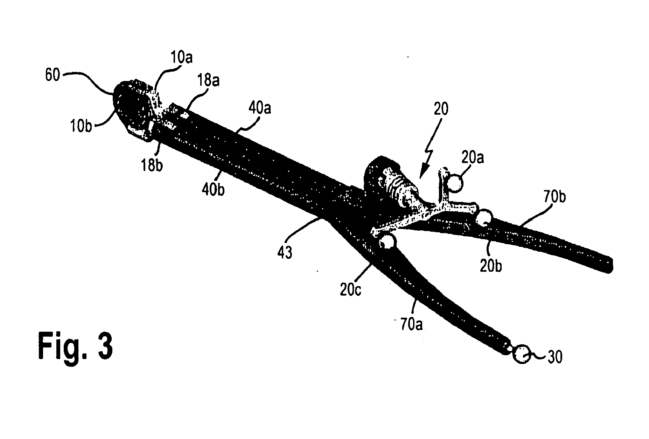

Fig. 3 zeigt eine weitere Ausführungsform eines erfindungsgemäßen Wirbelspreizinstruments. Funktionsgleiche Teile sind wiederum mit gleichen Bezugszeichen versehen.

Eine künstliche Bandscheibe ist mit 60 bezeichnet und wird durch die Spreizfinger 10a und 10b gehalten. Der Spreizfinger 10a ist in eine Ausnehmung 18a des Schenkels 40a eingesetzt. Entsprechend ist der Spreizfinger 10b in eine Ausnehmung 18b des Spreizfingers 40b eingesetzt. Die Spreizfinger sind also wiederum variabel je nach Anforderung austauschbar. Insbesondere können die Spreizfinger 10a und 10b unterschiedliche Aufnahmen für künstliche Bandscheiben aufweisen. Da die Spreizfinger 10a und 10b auswechselbar sind, können somit Bandscheiben beliebiger Form und Dicke eingesetzt werden. Wie bei der Ausführungsform in der Fig. 1 ergibt sich der Abstand der Spreizfinger wiederum aus der relativen Position zwischen dem Marker 30 und dem Referenzstern 20. Der Marker 30 befindet sich an einem Arm 70a, der mit dem Schenkel 40a starr verbunden ist und sich mit dem Schenkel 40a um ein Drehgelenk 43 dreht. Entsprechendes gilt für den Arm 70b, der mit dem Schenkel 40b starr verbunden ist und sich um dasselbe Drehgelenk 43 dreht. Ortsfest bezüglich des Drehgelenks 43 ist der Referenzstern 20 angeordnet. Durch Drehen der Arme 70a und 70b um das Drehgelenk 43 kann der Praktiker die Schenkel 40a und 40b auseinander bewegen und damit die Spreizfinger 10a und 10b. Durch das auseinander Bewegen der Arme 70a und 70b verändert sich die Relativposition des Markers 30 zu dem Referenzstern 20. Diese Veränderung ist eine Funktion des Abstandes zwischen den Spreizfingern 10a und 10b. Durch Vermessen der Marker 20a, 20b, 20c und 30 lässt sich somit der Abstand der Spreizfinger 10a und 10b genau bestimmen.

Die in Fig. 2 und 3 gezeigten Ausführungsformen erlauben somit durch eine Bestimmung der Relativposition des Referenzsterns 20 zum Marker 30 eine Bestimmung des Abstands zwischen den Spreizfingern 10a und 10b. Dadurch ist insbesondere eine Bestimmung des Abstandes der Spreizfinger 10a durch 10b durch Einlegen von Lehren in den Zwischenraum zwischen den Wirbeln nicht mehr nötig und der Eingriff wird somit erleichtert.

## Patentansprüche

1. Wirbelspreizinstrument zum Auseinanderbringen zweier Wirbel einer Wirbelsäule mittels zwei Spreizfingern (10a, 10b);
mit einem Spreizmechanismus (42, 43, 44, 50; 40, 43, 70) zum Verstellen des Abstandes zwischen den Spreizfingern, wobei der Spreizmechanismus einen ersten (45; 43) und zweiten Teil (50; 70a) aufweist und so ausgebildet ist, dass
die Teile (45, 50; 43, 70a) eine Veränderung ihrer relativen Positionen zueinander erfahren, wenn der Abstand verstellt wird, und
verschiedene relative Positionen verschiedenen Abständen eindeutig zugeordnet sind; wobei
der erste Teil (45; 43) einen ersten Marker (20a) aufweist und der zweite Teil (50; 70a) einen zweiten Marker (30) aufweist, **dadurch gekennzeichnet, dass** mindestens zwei weitere Marker (20b, 20c) vorgesehen sind, wobei die Marker Detektionsstrahlen oder Detektionswellen reflektieren oder emittieren.

2. Wirbelspreizinstrument nach Anspruch 1, bei welchem ein Referenzstern (20) den ersten Marker (20a) und mindestens zwei (20b, 20c) der mindestens zwei weiteren Marker umfasst und an dem ersten Teil (45; 43) vorgesehen ist.

3. Wirbelspreizinstrument nach einem der Ansprüche 1 bis 2, bei welchem der erste Teil (45; 43) ein Abschnitt des Spreizmechanismus ist, der durch Verstellung des Abstandes zwischen den Spreizfingern keine Lageänderung erfährt.

4. Wirbelspreizinstrument nach einem der Ansprüche 1 bis 3, bei welchem sich der zweite Teil (50; 70a) proximal vom ersten Teil (45; 43) befindet.

5. Wirbelspreizinstrument nach einem der Ansprüche 1 bis 4, bei welchem der Spreizmechanismus so ausgebildet ist, dass der zweite Teil (50) bei der Verstellung des Abstandes der Spreizfinger einen geradlinigen Weg zurücklegt, dessen Länge eine Funktion der Abstandsänderung ist.

6. Wirbelspreizinstrument nach einem der Ansprüche 1 bis 5, bei welchem ein Scheibenhalter vorgesehen ist, der zum Halten einer künstlichen Bandscheibe ausgebildet ist.

7. Wirbelspreizinstrument nach Anspruch 6, bei welchem der Scheibenhalter so ausgebildet ist, dass die Position einer gehaltenen künstlichen Bandscheibe mittels des Scheibenhalters relativ zu den Spreizfingern veränderbar ist, wobei der Scheibenhalter mindestens einen Marker aufweist, der eine feststehende relative Position zu einer angebrachten künstlichen Bandscheibe aufweist.

8. Instrumentennavigationssystem mit einem Wirbelspreizinstrument nach einem der Ansprüche 1 bis 7 und Detektionsmitteln zur Detektion von Detektionsstrahlen bzw. -wellen, die von den Markern ausgehen, wobei die Detektionsmittel so angeordnet sind, dass sie die Lage der Marker im Raum bestimmen können.

## Claims

1. A vertebral spreading instrument for moving apart two vertebrae of a spine, by means of two spreading fingers (10a, 10b);
comprising a spreading mechanism (42, 43, 44, 50; 40, 43, 70) for adjusting the distance between the spreading fingers, wherein the spreading mechanism comprises a first (45; 43) and a second part (50; 70a) and is designed such that
the parts (45, 50; 43, 70a) experience a change in their positions relative to each other when the distance is adjusted, and
different relative positions are unequivocally assigned to different distances;
wherein
the first part (45; 43) comprises a first marker (20a) and the second part (50; 70a) comprises a second marker (30), **characterised in that**
at least two other markers (20b, 20c) are provided, wherein the markers reflect or emit detection beams or detection waves.

2. The vertebral spreading instrument according to claim 1, wherein a reference star (20) comprises the first marker (20a) and at least two (20b, 20c) of the at least two other markers, and is provided on the first part (45; 43).

3. The vertebral spreading instrument according to any one of claims 1 to 2, wherein the first part (45; 43) is a section of the spreading mechanism which does not experience a change in position when the distance between the spreading fingers is adjusted.

4. The vertebral spreading instrument according to any one of claims 1 to 3, wherein the second part (50; 70a) is situated proximal to the first part (45; 43).

5. The vertebral spreading instrument according to any one of claims 1 to 4, wherein the spreading mechanism is designed such that the second part (50) travels a path in a straight line when the distance between the spreading fingers is adjusted, the length of said path being a function of the change in distance.

6. The vertebral spreading instrument according to any one of claims 1 to 5, wherein a disc holder is provided which is designed for holding an artificial intervertebral disc.

7. The vertebral spreading instrument according to claim 6, wherein the disc holder is designed such that the position of an artificial intervertebral disc held can be changed relative to the spreading fingers by means of the disc holder, and the disc holder comprises at least one marker which exhibits a fixed position relative to an attached artificial intervertebral disc.

8. An instrument navigation system comprising a vertebral spreading instrument according to any one of claims 1 to 7 and comprising detection means for detecting detection beams or waves emitted by the markers, wherein the detection means are arranged such that they can determine the spatial position of the markers.

## Revendications

1. Ecarteur vertébral pour écarter deux vertèbres d'une colonne vertébrale au moyen de deux doigts d'écartement (10a, 10b) ;
avec un mécanisme d'écartement (42, 43, 44, 50 ; 40, 43, 70) pour régler la distance entre les doigts d'écartement, le mécanisme d'écartement comportant une première partie (45 ; 43) et une seconde partie (50 ; 70a) et étant réalisé de manière que
les parties (45, 50 ; 43, 70a) subissent une variation de leurs positions relatives, lorsque la distance est réglée, et
différentes positions relatives soient clairement corrélées à différentes distances ;
dans lequel
la première partie (45 ; 43) présente un premier repère (20a) et la deuxième partie (50 ; 70a) un second repère (30), **caractérisé en ce que**
il est prévu au moins deux autres repères (20b, 20c), les repères réfléchissant ou émettant des rayons de détection ou des ondes de détection.

2. Ecarteur vertébral selon la revendication 1, dans lequel une étoile de référence (20) comprend le premier repère (20a) et au moins deux repères (20b, 20c) des au moins deux autres repères, et est prévue sur la première partie (45 ; 43).

3. Ecarteur vertébral selon l'une des revendications 1 ou 2, dans lequel la première partie (45 ; 43) est un segment du mécanisme d'écartement qui ne subit aucune variation de position, par réglage de la distance entre les doigts d'écartement.

4. Ecarteur vertébral selon l'une des revendications 1 à 3, dans lequel la seconde partie (50 ; 70a) se trouve sur le côté proximal de la première partie (45 ; 43).

5. Ecarteur vertébral selon l'une des revendications 1 à 4, dans lequel le mécanisme d'écartement est réalisé de manière que lors du réglage de la distance des doigts d'écartement, la seconde partie (50) franchisse une course rectiligne dont la longueur est fonction de la variation de distance.

6. Ecarteur vertébral selon l'une des revendications 1 à 5, dans lequel il est prévu un porte-disque qui est réalisé pour soutenir un disque intervertébral artificiel.

7. Ecarteur vertébral selon la revendication 6, dans lequel le porte-disque est réalisé de manière que la position d'un disque intervertébral artificiel maintenu soit variable au moyen du porte-disque, par rapport aux doigts d'écartement, le porte-disque présentant au moins un repère qui présente une position relative fixe par rapport à un disque intervertébral artificiel mis en place.

8. Système de navigation d'instrument avec un écarteur vertébral selon l'une des revendications 1 à 7 et moyens de détection pour détecter des rayons ou ondes de détection qui partent des repères, les moyens de détection étant disposés de manière à pouvoir déterminer la position des repères dans l'espace.
